(19) Europäisches Patentamt
European Patent Office
Office européen des brevets

(11) EP 0 742 693 B1

(12) **EUROPÄISCHE PATENTSCHRIFT**

(45) Veröffentlichungstag und Bekanntmachung des Hinweises auf die Patenterteilung:
**26.11.1997 Patentblatt 1997/48**

(21) Anmeldenummer: **95909788.2**

(22) Anmeldetag: **27.02.1995**

(51) Int. Cl.$^6$: **A61B 5/22**

(86) Internationale Anmeldenummer:
**PCT/EP95/00711**

(87) Internationale Veröffentlichungsnummer:
**WO 95/22929 (31.08.1995 Gazette 1995/37)**

# (54) BESTIMMUNG DES INDIVIDUELLEN ANAEROBEN SCHWELLWERTES

DETERMINATION OF THE INDIVIDUAL ANAEROBIC THRESHOLD

DETERMINATION DE LA VALEUR LIMITE INDIVIDUELLE D'ANAEROBIOSE

(84) Benannte Vertragsstaaten:
**AT BE CH DE DK ES FR GB GR IE IT LI LU MC NL PT SE**

(30) Priorität: **26.02.1994 DE 4406286**

(43) Veröffentlichungstag der Anmeldung:
**20.11.1996 Patentblatt 1996/47**

(73) Patentinhaber: **Stegmann, Heiner**
**63450 Hanau (DE)**

(72) Erfinder: **Stegmann, Heiner**
**63450 Hanau (DE)**

(74) Vertreter:
**Stoffregen, Hans-Herbert, Dr. Dipl.-Phys.**
**Patentanwalt**
**Postfach 21 44**
**63411 Hanau (DE)**

(56) Entgegenhaltungen:
**US-A- 3 675 640**

- **MEDICAL & BIOLOGICAL ENGINEERING & COMPUTING, Bd.23, Nr.6, November 1985, STEVENAGE (GB) Seiten 579 - 584 T.L. TALBOT ET AL. 'Noninvasive detection of the anaerobic threshold during computer-controlled exercise testing'**
- **JOURNAL OF APPLIED PHYSIOLOGY (US), Bd.64, Nr.1, 1988 Seiten 50 - 60 J.I. MEDBO ET AL. 'Anaerobic capacity determined by maximal accumulated O2 deficit'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.14.23/3.4

EP 0 742 693 B1

**Beschreibung**

Die Erfindung bezieht sich auf eine Vorrichtung zur dynamischen Erfassung der respiratorischen, metabolischen und/oder ventilatorischen Größen während eines die Lactatproduktion fördernden Vorganges oder Einflusses, mit einer eine Anzeigeeinheit und eine Recheneinheit aufweisenden Datenverarbeitungsanlage sowie einer über ein Analysegerät mit der Datenverarbeitungsanlage verbundenen Einrichtung wie Maske zur Bestimmung des Atemminutenvolumens sowie des $O_2$-Gehalts und $CO_2$-Gehalts des Atemminutenvolumens der aufgenommenen bzw. abgegebenen Luft.

Ferner bezieht sich die Erfindung auf ein Verfahren zur reproduzierbaren Beurteilung körperlicher Leistungsfähigkeit eines Lebewesens wie Menschens während eines die Lactatproduktion fördernden Vorganges oder Einflusses durch Bestimmung der "individuellen anaeroben Schwelle".

Bei der "individuellen anaeroben Schwelle" (Lactate Kinetics and Individual Anaerobic Threshold, Stegmann et al. International Journal of Sports Medicine, S. 160 - 165, Georg Thieme Verlag Stuttgart, 1981) handelt es sich um einen festen, eindeutig reproduzierbaren Wert. Dabei ist die "individuelle anaerobe Schwelle" auch für physiologische Parameter wie Beurteilung der körperlichen Leistungsfähigkeit, Belastungsblutdruck und koronale Herzerkrankung von Bedeutung.

Nach dem Stand der Technik wird die "individuelle anaerobe Schwelle" durch Laktatbestimmung, also durch Milchsäurebestimmung im Blut vorgenommen. Man bestimmt die Änderung des Milchsäureanteils in Abhängigkeit von der pro Zeiteinheit geleisteten Arbeit.

Nachteil dieses Verfahrens ist es, daß fortwährend der Person, von der die individuelle anaerobe Schwelle bestimmt werden soll, Blut vorzugsweise aus dem Ohrläppchen, entnommen werden muß. Solche Maßnahmen sind nicht nur umständlich, sondern ermöglichen auch nur zeitversetzte Bestimmungen der "individuellen anaeroben Schwelle".

Aus dem Stand der Technik ist des weiteren bekannt, daß Atemäquivalent

$$O_2 \left(\frac{\dot{V}_e}{\dot{V}_{O_2}}\right)$$

sowie das Atemäquivalent

$$CO_2 \left(\frac{\dot{V}_e}{\dot{V}_{CO_2}}\right),$$

wobei die Ventilationsparameter durch eine

Vorrichtung der oben genannten Art ermittelt werden, über die Zeit aufgetragen werden. Dabei wurde die "anaerobe Schwelle" als der Punkt definiert, in dem ein starker Anstieg des Atemäquivalents $O_2$ registriert wird, ohne daß die Kurve für das Atemäquivalent $CO_2$ ansteigt. Zur Bestimmung der "anaeroben Schwelle" ist jedoch weiterhin eine Lactatbestimmung des Blutes der belasteten Person notwendig (H.A. Davis and G.C. Gass: The anaerobic threshold as determined before and during lactic Acidosis, European Journal of Applied Physiology, Springer-Verlag 1981).

Des weiteren wird vorgeschlagen, eine bestimmte Lactatkonzentration vorzugeben (J.A. Davis u.a.: Does the gas exchange anaerobic threshold occur at a fixed blood lactate concentration of 2 or 4 m/M4?, International Journal Sports Medicin 4 (1983), S. 89 - 93, Georg Thieme Verlag Stuttgart). Dabei wird wiederum das Atemäquivalent

$$O_2 \left(\frac{\dot{V}_e}{\dot{V}_{O_2}}\right)$$

sowie das Atemäquivalent

$$CO_2 \left(\frac{\dot{V}_e}{\dot{V}_{CO_2}}\right)$$

gemessen und ein systematischer Anstieg des

Atemäquivalents $O_2$ als "anaerobe Schwelle" definiert, wenn das Atemäquivalent $CO_2$ an dieser Stelle keinen merklichen Anstieg aufweist. Diese Auswertung ist jedoch mit großen Ungenauigkeiten verbunden und erlaubt nicht die Bestimmung der "individuellen anaeroben Schwelle".

Der vorliegenden Erfindung liegt das Problem zugrunde, die Bestimmung der individuellen anaeroben Schwelle eines Menschen zu ermöglichen, ohne daß fortwährend eine Lactatbestimmung durch Blutentnahme erfolgen muß. Dabei sollte die individuelle anaerobe Schwelle mit hoher Genauigkeit bestimmt werden können.

Das Problem wird erfindungsgemäß dadurch gelöst, daß die zuvor beschrieben Vorrichtung zur Bestimmung der individuellen anaeroben Schwelle verwendet wird, wobei in der Recheneinheit aus den respiratorischen Meßgrößen des Atemminutenvolumens $\dot{V}_e$, des $O_2$-Gehalts des Atemminutenvolumens

$$\dot{V}_{O_2}$$

und des

$$C_{O_2}$$

-Gehalts des Atemminutenvolumens

$$\dot{V}_{CO_2}$$

nach einem Algorhythmus

$$x = \frac{\dot{V}_e}{\sqrt[3]{(\dot{V}^2_{CO_2}) \cdot \dot{V}_{O_2}}}$$

eine Größe x in Abhängigkeit von der pro Zeiteinheit geleisteten Arbeit (P) bestimmbar ist, daß an die Werte x = f(P) eine Kurve angepaßt oder eine solche durch diese gelegt wird und wobei ein der individuellen anaeroben Schwelle entsprechender Belastungswert PIAT einen Punkt der Kurve x = f(P) bestimmt, an dem die Ableitung dx(P) / dP null ist oder eine maximale Unstetigkeit aufweist.

Erfindungsgemäß hat sich überraschenderweise gezeigt, daß allein das Atemminutenvolumen sowie die $CO_2$- und $O_2$-Gehalte dieser ermittelt werden müssen, um aus diesen Werten unmittelbar die individuelle anaerobe Schwelle zu bestimmen, ohne daß es folglich einer fortwährenden Lactatbestimmung bedarf.

Der vorliegenden Erfindung liegt der Gedanke zugrunde, daß die $CO_2$-Sättigung SAT ($CO_2$) sich proportional dem Quadrat des Partialdrucks von $CO_2$ p ($CO_2$) und die $O_2$-Sättigung SAT ($O_2$) sich linear zu dem Partialdruck $O_2$ p ($O_2$) verhält. Auch wird ausgenutzt, daß die Proportionalitäten bei Bildung von Lactat einer starken Veränderung ausgesetzt sind.

Erfindungsgemäß werden die ermittelten Atemminutenvolumina und deren $O_2$- und $CO_2$-Gehalte korreliert, um nach der Beziehung

$$x = \frac{\dot{V}_e}{\sqrt[3]{(\dot{V}^2_{CO_2}) \cdot \dot{V}_{O_2}}}$$

eine Größe x (als Atemäquivalent für Gasdruck) zu erhalten, die an ihrem Minimum bzw. finalen Anstieg, oder mathematisch ausgedrückt an einem Punkt, an dem die zeitliche Ableitung dx(t) / dt null ist oder eine maximale Unstetigkeit aufweist, einen der individuellen anaeroben Schwelle entsprechenden Belastungswert PIAT (Punkt der Individuellen Anaeroben Schwelle (Threshold)) angibt bzw. definiert.

Die Bestimmung der Änderung der Parameter kann dabei in Abhängigkeit von einer stufenweisen ansteigenden Betätigung des Probanden erfolgen. Selbstverständlich besteht auch die Möglichkeit, daß eine kontinuierliche Leistungssteigerung beobachtet wird.

Es kann jedoch auch jeder weitere Vorgang, der zu einer Lactatkkumulation im Blut führt, zur Bestimmung der individuellen anaeroben Schwelle, also der Schwellenleistung, herangezogen werden. Als Beispiel ist anzugeben Sauerstoffentzug, Intoxikation durch Medikamente, Reduzierung der Herzleistung oder Lactatinfusion.

Allgemein wird daher erfindungsgemäß auch ein Verfahren zur reproduzierbaren Beurteilung körperlicher Leistungsfähigkeit eines Lebewesens wie Menschens während eines die Lactatproduktion fördernden Vorganges oder Einflusses durch Bestimmung der individuellen anaeroben Schwelle vorgeschlagen wobei das Atemminutenvolumen ($\dot{V}_e$) sowie der $O_2$- und $CO_2$-Gehalt des Atemminutenvolumens

$$(\dot{V}_{O_2}; \dot{V}_{CO_2})$$

Gemessen werden, das sich dadurch auszeichnet, daß in Abhängigkeit des die Lactatproduktion fördernden Vorgangs oder Einflusses aus den gemessenen Werten des Atemminutenvolumens $\dot{V}_e$ sowie des $O_2$- und $CO_2$-Gehalts des Atemminutenvolumens

$$(\dot{V}_{O_2}; \dot{V}_{CO_2})$$

eine Größe x nach dem Algorhythmus aus

$$x = \frac{\dot{V}_e}{\sqrt[3]{(\dot{V}^2_{CO_2}) \cdot \dot{V}_{O_2}}}$$

in Abhängigkeit von der Zeit bestimmt wird, in der der die Lactatproduktion fördernde Einfluß oder Vorgang auf das Lebewesen einwirkt, und daß an die Werte x = f(t) eine Kurve angepaßt oder eine solche durch diese gelegt wird, wobei ein der individuellen anaeroben Schwelle entsprechender Wert (PIAT) durch einen Punkt der Kurve bestimmt wird, an dem die zeitliche Ableitung dx(t) / d(t) null ist oder eine maximale Unstetigkeit aufweist.

Weitere Einzelheiten, Vorteile und Merkmale der Erfindung ergeben sich nicht nur aus den Ansprüchen, den diesen zu entnehmenden Merkmalen - für sich und/oder in Kombination-, sondern auch aus der nachfolgenden Beschreibung eines den Zeichnungen zu entnehmenden Ausführungsbeispiels.

Es zeigen:

Fig. 1 eine Versuchsanordnung zur Bestimmung der individuellen anaeroben Schwelle und

Fig. 2 einen Graphen zur Bestimmung der individuellen anaeroben Schwelle und

Fig. 3 eine Vielzahl von Graphen zur Bestimmung der individuellen anaeroben Schwellen bei unterschiedlichen Belastungsarten.

Um die individuelle anaerobe Schwelle eines Probanden zu ermitteln, muß dieser über einen Zeitraum Arbeit leisten. Parallel wird das Atemminutenvolumen $\dot{V}_e$ sowie der $CO_2$- und $O_2$-Gehalt von dem Probanden bestimmt.

Um die Belastungsmessung durchzuführen, kann ein Fahrradergometer (10) oder alternativ zum Beispiel ein Laufband (12) benutzt werden. Die entsprechenden Geräte (10) bzw. (12) sind über Leitungen (14) und (16) mit einer Datenverarbeitungsanlage (18) verbunden, um so die pro Zeiteinheit geleistete Arbeit zu ermitteln.

Um das Atemminutenvolumen bzw. dessen $CO_2$ und $O_2$-Gehalt zu ermitteln, wird dem Probanden eine Maske (20) oder Mundstück aufgesetzt, die rein schematisch in Fig. 1 dargestellt wird.

Die von dem Probanden aufgenommene Luft bzw. der in der abgegebenen Luft vorhandene $O_2$- bzw. $CO_2$-Gehalt wird über ein Analysegerät (22) bestimmt, um die Werte sodann der Datenverarbeitungsanlage (18) über Leitungen (24) und (26) zuzuführen.

Aus den so gemessenen bzw. ermittelten Werten, also aus geleisteter Arbeit pro Zeiteinheit, Atemminutenvolumen $\dot{V}_e$ sowie $CO_2$- und $O_2$-Verbrauch von diesem wird sodann nach der Beziehung

$$x = \frac{\dot{V}_e}{\sqrt[3]{(\dot{V}^2_{CO_2}) \cdot \dot{V}_{O_2}}}$$

der Wert x bestimmt und über die Zeit bzw. die Leistung aufgetragen, um Meßpunkte zu erhalten, die in den Fig. 2 und 3 dargestellt sind.

Ein Meßvorgang sei anhand des Graphen $x = f(t)$ erläutert. Nach einer Zeiteinheit $\Delta t$ (hier $\Delta t$= 1 min.) und stufenweise erfolgter Leistungserhöhung $\Delta p$ (hier $\Delta p$ = 50 W) werden pro Zeiteinheit Meßwerte x über die Zeit t aufgetragen. Nach durchgeführter Messung werden die Meßpunkte durch eine Funktion $x = f(t)$, die rechnerunterstützt ermittelt werden kann, beschrieben. Mit anderen Worten werden die Meßkurven dadurch bestimmt, daß für jede Zeiteinheit, in der die Belastung um einen konstanten Wert erhöht wird, der Wert $x = f(t)$ nach der zuvor genannten Gleichung ermittelt wird.

Die Auswertung der Kurve $x = f(t)$ erfolgt durch Bildung der zeitlichen Ableitung. Der Punkt der Kurve, in dem die zeitliche Ableitung 0 oder eine maximale Unstetigkeit aufweist, entspricht einem der individuellen anaeroben Schwelle entsprechenden Belastungswert PIAT.

Fig. 3 zeigt verschiedene Meßkurven (28), (30), (32), (34), die sich dadurch unterscheiden, daß sich das Zeitintervall der Belastungsänderung $\Delta t$ von einer Zeiteinheit $\Delta t$ = 1 min. (Kurve 34) bis zu einer Zeiteinheit $\Delta t$ = 5 min. (Kurve 28) erhöht. Die pro Zeiteinheit erhöhte Leistung $\Delta p$ entspricht bei allen Meßkurven einem Wert von $\Delta p$ = 50 W.

Bei Belastung in einem Zeitintervall von $\Delta t \geq 1$ min. bis zum Beispiel $\Delta t$ = 5 min. kann festgestellt werden, daß für den Probanden, für den die Kurven (28), (30), (32) und (34 ermittelt wurden, eine individuelle anaerobe Schwelle von PIAT 175 ± 5 Watt ermittelt werden kann. Dies entspricht dem Punkt, bei dem bei einer Leistungsabgabe anaerobe Arbeit geleistet wird.

**Patentansprüche**

1. Vorrichtung zur dynamischen Erfassung der respiratorischen, metabolischen und/oder ventilatorischen Größen während eines die Lactatproduktion fördernden Vorgangs oder Einflusses, mit einer eine Anzeigeeinheit und eine Recheneinheit aufweisenden Datenverarbeitungsanlage (18) sowie einer über ein Analysegerät (22) mit der Datenverarbeitungsanlage verbundenen Einrichtung wie Maske (20) zur Bestimmung des Atemminutenvolumens sowie des $O_2$-Gehalts bzw. $CO_2$-Gehalts des Atemminutenvolumens der aufgenommenen bzw. abgegebenen Luft,
**dadurch gekennzeichnet**,
daß die Vorrichtung zur Bestimmung der individuellen anaeroben Schwelle verwendet wird, wobei in der Recheneinheit aus den respiratorischen Meßgrößen des Atemminutenvolumens $\dot{V}_e$, und des $O_2$-Gehalts des Atemminutenvolumens

$$\dot{V}_{O_2}$$

und des $CO_2$-Gehalts des Atemminutenvolumens

$$\dot{V}_{CO_2}$$

nach einem Algorhythmus

$$x = \frac{\dot{V}_e}{\sqrt[3]{(\dot{V}^2_{CO_2}) \cdot \dot{V}_{O_2}}}$$

eine Größe x in Abhängigkeit von der pro Zeiteinheit geleisteten Arbeit (P) bestimmt wird, daß an die Werte $x = f(P)$ eine Kurve angepaßt oder eine solche durch diese gelegt wird und wobei ein der individuellen anaeroben Schwelle entsprechender

Belastungswert (PIAT) einen Punkt der Kurve x = f(P) bestimmt, an dem die Ableitung dx(P) / dP null ist oder eine maximale Unstetigkeit aufweist.

**2.** Vorrichtung nach Anspruch 1, **dadurch gekennzeichnet**, daß die Leistungsänderung stufenweise einstellbar ist.

**3.** Vorrichtung nach Anspruch 1, **dadurch gekennzeichnet**, daß die Leistungsänderung kontinuierlich einstellbar ist.

**4.** Verfahren zur reproduzierbaren Beurteilung körperlicher Leistungsfähigkeit eines Lebewesens wie Menschens während eines die Lactatproduktion fördernden Vorganges oder Einflusses durch Bestimmung der individuellen anaeroben Schwelle, wobei das Atemminutenvolumen ($\dot{V}_e$) sowie der $O_2$- und $CO_2$-Gehalt des Atemminutenvolumens

$$(\dot{V}_{O_2};\dot{V}_{CO_2})$$

Gemessen werden **dadurch gekennzeichnet**, daß in Abhängigkeit des die Lactatproduktion fördernden Vorgangs oder Einflusses aus den gemessenen Werten des Atemminutenvolumens ($\dot{V}_e$) sowie des $O_2$- und $CO_2$-Gehalts des Atemminutenvolumens

$$(\dot{V}_{O_2};\dot{V}_{CO_2})$$

eine Größe x nach dem Algorhythmus

$$x = \frac{\dot{V}_e}{\sqrt[3]{(\dot{V}^2_{CO_2})\cdot \dot{V}_{O_2}}}$$

in Abhängigkeit von der Zeit bestimmt wird, in der der die Lactatproduktion fördernde Einfluß oder Vorgang auf das Lebewesen einwirkt, und daß an die Werte x = f(t) eine Kurve angepaßt oder eine solche durch diese gelegt wird, wobei ein der individuellen anaeroben Schwelle entsprechender Wert (PIAT) durch einen Punkt der Kurve bestimmt wird, an dem die zeitliche Ableitung dx(t) / d(t) null ist oder eine maximale Unstetigkeit aufweist.

**5.** Verfahren nach Anspruch 4, **dadurch gekennzeichnet**, daß der die Lactatproduktion fördernde Einfluß bzw. Vorgang durch von dem Lebewesen pro Zeiteinheit geleistete Arbeit, durch Lactatinfusion, durch Intoxikation von Medikamenten und/oder durch Reduzierung der Herzleistung herbeigeführt wird.

## Claims

**1.** A device for dynamic recording of the respiratory, metabolic and/or ventilatory quantities during a process or influence conducive to lactate production, having a data processing facility (18) incorporating a display unit and also a computing unit, and having a device such as a mask (20) connected via an analysis instrument (22) to said data processing facility for determination of the respiratory minute volume and also of the $O_2$ content or $CO_2$ content of said respiratory minute volume of the inhaled or exhaled air,
**wherein**
said device is used for determination of the individual anaerobic threshold, with said computer unit using the respiratory measuring quantities of respiratory minute

$$\dot{V}_{e1}$$

volume of the $O_2$ content of respiratory minute volume

$$\dot{V}_{O_2}$$

and of the $CO_2$ content of respiratory minute volume

$$\dot{V}_{CO_2}$$

according to an algorithm

$$x = \frac{\dot{V}_e}{\sqrt[3]{(\dot{V}^2_{CO_2})_{\dot{V}_{O_2}}}}$$

to permit determination of a quantity x as a function of the work performed per unit of time (P), wherein a curve is adapted to the values x = f(P) or a curve is passed through these values, with a load value (PIAT) corresponding to said individual anaerobic threshold determining a point of the curve x = f(P) at which the derivation dx(P) / dP is zero or has a maximum discontinuity.

2. A device according to Claim 1, **wherein** the change in performance can be set gradually.

3. A device according to Claim 1, **wherein** the change in performance can be set continuously.

4. A method for reproducible assessment of the physical capacity of a living organism such as a human being during a process or influence conducive to lactate production by determination of the individual anaerobic threshold, wherein the respiratory minute volume ($\dot{V}_e$) as well as the $O_2$ and $CO_2$ contents of respiratory minute volume

$$(\dot{V}_{O_2}; \dot{V}_{CO_2})$$

are measured) **wherein** as a function of said process or influence conducive to lactate production from the measured values of the respiratory minute volume ($\dot{V}e$) and of the $O_2$ and $CO_2$ contents of said respiratory minute volume

$$(\dot{V}_{O_2}; \dot{V}_{CO_2})$$

a quantity x is determined on the basis of the algorithm

$$x = \frac{\dot{V}_e}{\sqrt[3]{(\dot{V}^2_{CO_2})_{\dot{V}_{O_2}}}}$$

as a funtion of the time in which said influence or process conductive to lactate production acts on the living organism, and wherein a curve is adapted to or passes through the values $x = f(t)$, with a value (PIAT) corresponding to said individual anaerobic threshold being determined by a point on the curve at which the time derivation $dx(t) / d(t)$ is zero or has a maximum discontinuity.

5. A method according to Claim 4, **wherein** said influence or process conducive to lactate production is caused by the work performed by the living organism per unit of time, by lactate infusion, by intoxication by medicines and/or reduction of the heart output.

**Revendications**

1. Dispositif pour la détermination ou l'enregistrement dynamique des grandeurs respiratoires, métaboliques et/ou relatives à la ventilation au cours d'une influence sur ou d'un processus faisant progresser la production de lactate, avec une installation de traitement de données (18) comprenant une unité d'affichage et une unité de calcul, ainsi qu'à l'aide d'un appareil d'analyse (22) avec le dispositif raccordé à l'installation de traitement de données, comme des masques (20) pour la détermination du volume-minute respiratoire, ainsi que de la teneur en $O_2$ ou de la teneur en $CO_2$ du volume-minute respiratoire de l'air inspiré et expiré, caractérisé en ce que le dispositif est utilisé pour la détermination des seuils anaérobies individuels où, dans l'unité de calcul, à partir des grandeurs respiratoires mesurées du volume-minute respiratoire $\dot{V}_e$, de la teneur en $O_2$ du volume-minute respiratoire

$$\dot{V}_{O_2}$$

et de la teneur en $CO_2$ du volume-minute respiratoire

$$\dot{V}_{CO_2},$$

on détermine, selon un algorithme

$$x = \frac{\dot{V}_e}{\sqrt[3]{(\dot{V}^2_{CO_2}) \cdot \dot{V}_{O_2}}}$$

qu'une grandeur x en fonction du travail fourni par unité de temps (P), en ce qu'à la valeur $x = f(P)$ on adapte une courbe ou on établit une telle courbe imposée par ce travail et où on détermine un point de la courbe $x = f(P)$, la valeur de la charge (PLAT) correspondant à l'un des seuls anaérobies individuels, auquel la dérivée $dx(P) / dP$ est nulle ou présente une discontinuité maximale.

2. Dispositif suivant la revendication 1, caractérisé en ce que la modification de puissance peut être réglée de manière échelonnée.

3. Dispositif suivant la revendication 1, caractérisé en ce que la modification de puissance peut être réglée de manière continue.

4. Procédé pour la détermination reproductible de la capacité de puissance corporelle d'un être vivant,

comme l'homme, au cours d'une influence sur ou d'un processus faisant progresser la production de lactate par la détermination des seuils anaérobies individuels où l'on mesure le volume-minute respiratoir ($\dot{V}_e$), ainsi que la teneur en $O_2$ et en $CO_2$ du volume-minute respiratoire

$$(\dot{V}_{O_2} ; V_{CO_2}),$$

caractérisé en ce que,
en fonction de l'influence sur ou du processus faisant progresser la production de lactate, on détermine, à partir des valeurs mesurées du volume-minute respiratoire ($\dot{V}_e$), ainsi que de la teneur en $O_2$ et en $CO_2$ du volume-minute respiratoire

$$(\dot{V}_{O_2} ; \dot{V}_{CO_2}),$$

une grandeur x, selon l'algorithme

$$x = \frac{\dot{V}_e}{\sqrt[3]{(\dot{V}^2_{CO_2}) \cdot \dot{V}_{O_2}}}$$

en fonction de la durée pendant laquelle l'influence sur ou le processus faisant progresser la production de lactate agit sur l'être vivant et en ce que aux valeurs $x = f(t)$ on adapte une courbe ou on établit une telle courbe imposée par ce travail et où on détermine un point de la courbe $x = f(t)$, la valeur de la charge (PLAT) correspondant à l'un des seuils anaérobies individuels, auquel la dérivée $dx(t) / dt$ est nulle ou présente une discontinuité maximale.

**5.** Procédé suivant la revendication 4, caractérisé en ce que l'influence sur ou le processus faisant progresser la production de lactate, en ce que le travail fourni par unité de temps par l'être vivant pour l'influence sur ou le processus faisant progresser la production de lactate est entraîné par perfusion de lactate, par intoxication par des médicaments et/ou par réduction du débit cardiaque.

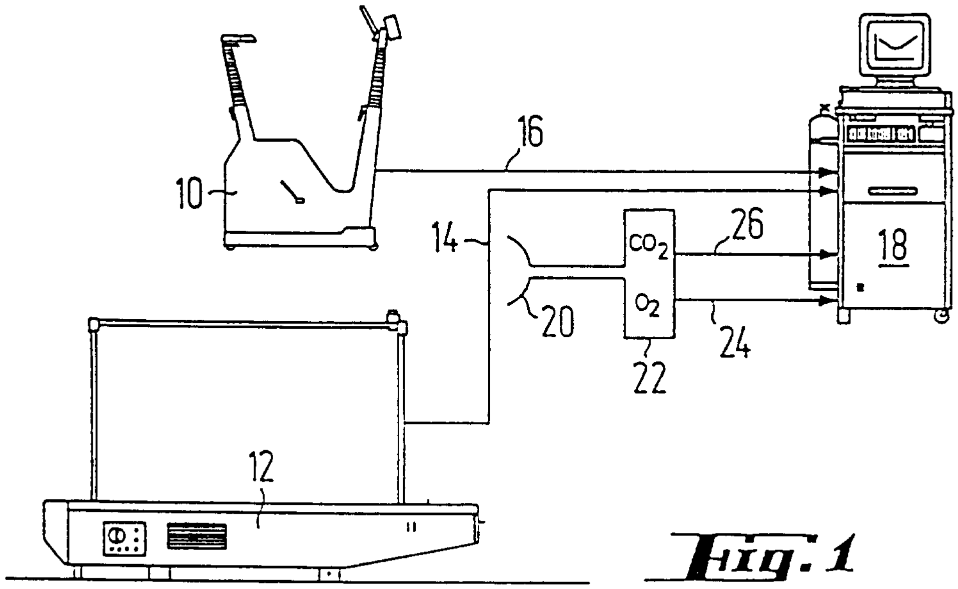
10
12
14
16
$CO_2$
$O_2$
18
20
22
24
26

Fig. 1

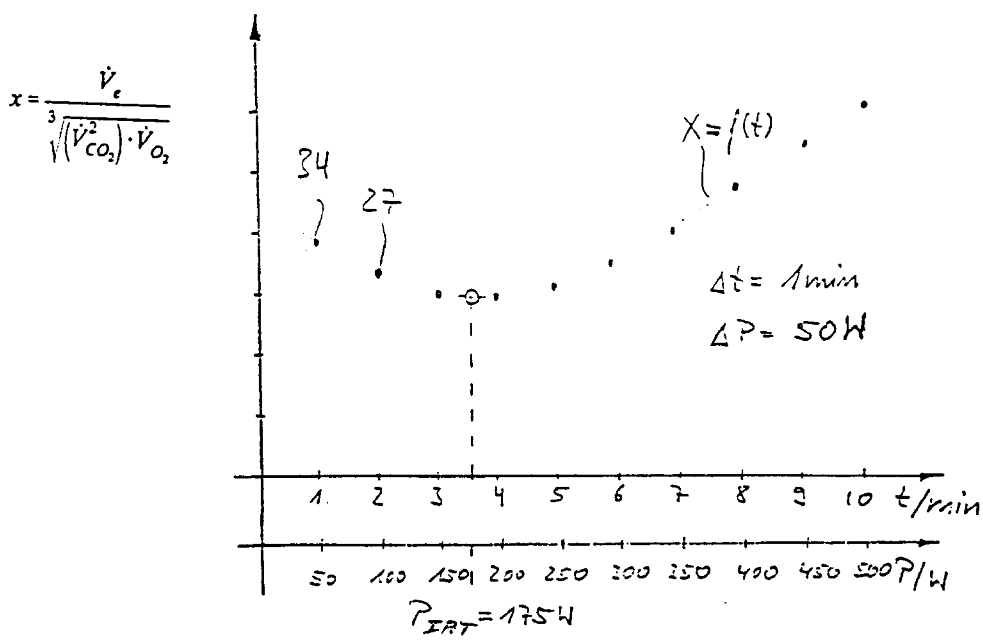
$x = \frac{\dot{V}_e}{\sqrt[3]{(\dot{V}^2_{CO_2}) \cdot \dot{V}_{O_2}}}$
34
27
X = f(t)
Δt = 1min
ΔP = 50W
1 2 3 4 5 6 7 8 9 10 t/min
50 100 150 200 250 300 350 400 450 500 P/W
$P_{IAT}$ = 175W

Fig. 2

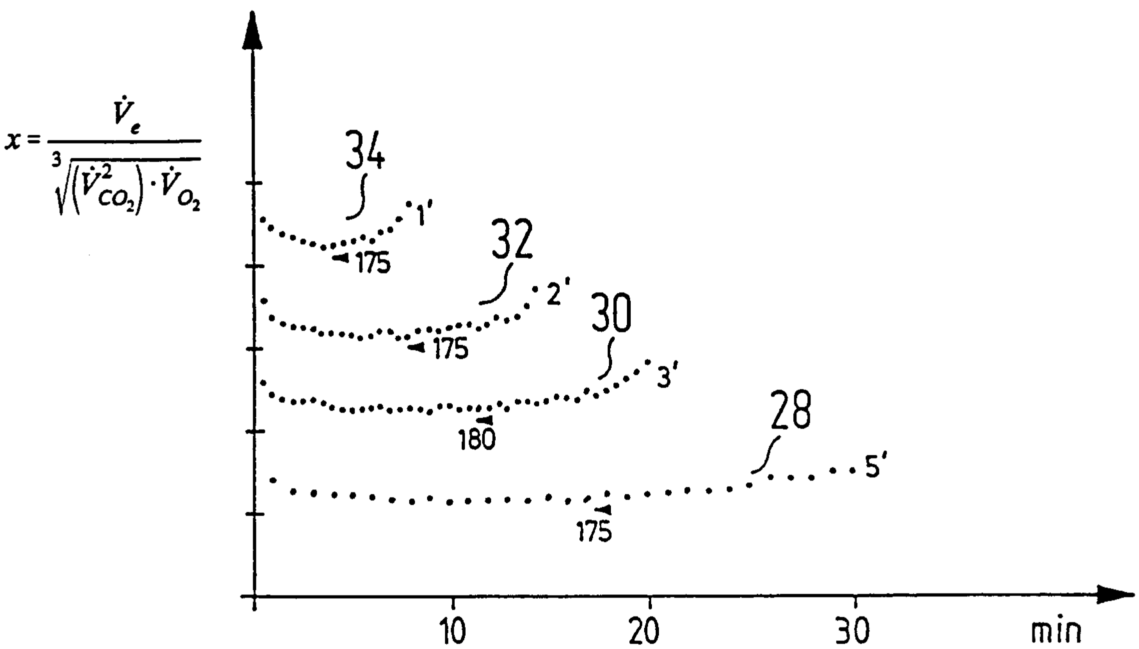
$x = \frac{\dot{V}_e}{\sqrt[3]{(\dot{V}^2_{CO_2}) \cdot \dot{V}_{O_2}}}$
34
1'
175
32
2'
175
30
3'
180
28
5'
175
10
20
30
min

Fig. 3